# EUROPEAN PATENT SPECIFICATION

(11) **EP 0 794 730 B1**
(45) Date of publication and mention of the grant of the patent: **19.05.1999**
(21) Application number: 95938504.8
(22) Date of filing: 29.11.1995
(51) Int. Cl.: A61B 5/087, A61B 5/09

(54) **PEAK-FLOW METER**
DURCHFLUSSMESSER ZUM ANZEIGEN VON HÖCHSTWERTEN
DISPOSITIF DE MESURE DU DEBIT DE POINTE

(30) Priority: 30.11.1994 GB 9424230
(43) Date of publication of application: 17.09.1997
(73) Proprietor: Sovereign Surgical Limited, Basildon, Essex SS14 3BR (GB)
(72) Inventor: FORD, Thomas McDonald, Sovereign Surgical Limited, Basildon, Essex SS14 3BR (GB)
(74) Representative: Jones, William
(86) International application number: PCT/GB95/02793
(87) International publication number: WO 96/16598

(56) References cited:
- DE-A- 1 803 325
- FR-A- 2 106 452
- BIOMEDIZINISCHE TECHNIK, vol. 21, no. 8, October 1976 BERLIN (DDR), pages 228-230, R. SCHNEIDERREIT 'Das Wirbelrespirometer- ein Gerät zum Erfassen der Atemstromgeschwindigkeit in beiden Atemphasen nach dem Flügelradsystem'

## Description

### Field of the Invention

This invention relates to the field of apparatus for measuring lung function.

### Background to the Invention

During normal adult respiration, the amount of air inspired or expired per breath (tidal air) is approximately 500ml.

After a normal tidal expiration, a further volume of air (the expiratory reserve-volume) can be expired - approximately 750-1000ml in adults.

Vital capacity is the maximum volume of air which can be expelled from the lungs by a forceful effort, following a deliberate inhalation of the maximum volume possible. This vital capacity is normally of the order of 4.8 litres in men and 3.2 litres in women, but can be substantially reduced in asthmatics and people with other bronchial problems. The vital capacity and/or the ability to exhale thus provides a good indication of a subject's lung function.

Several devices exit which monitor a subject's ability to exhale, thus providing an indication of lung function. These devices employ a number of different methods which either directly or indirectly provide a measurement of expiratory volume; tidal reserve or more preferably vital capacity. For example, a subject may be asked to blow into a calibrated tube containing a spring-loaded device, for example a piston, which is moved along a calibrated scale according to the force provided by the exhaled air. The inertia of the spring-loaded device will affect the sensitivity of the equipment and may require that, for a valid measurement, a degree of force is needed which a severe asthmatic, or otherwise respiratory-impaired individual, cannot provide.

An alternative method involves the use of a device containing a hot wire. Exhaled air passes over the hot wire, cooling it and a measure of the degree of cooling thus provides an indication of lung function. Again the device may not be sensitive enough for some subjects. In addition the difficulty in accurately measuring the degree of cooling further affects the sensitivity.

A further method involves the use of rotating blades or a turbine. Exhaled air causes the blades to rotate and this motion is detected and translated into an indication of lung function. The device is generally arranged so that the exhaled air impinges on the edges of the blades (i.e perpendicular to their mounting) and, again, the inertia of the device will affect its sensitivity, particularly in subjects with poor lung function.

DE-A-1803325 describes a breath volumeter having a rotor fitted with a twin-armed vane arranged centrally below a semi-circular-shaped stator attached to the meter casing. The stator has a roughly semi-circular cross-section. In use, this arrangement suffers from the disadvantage of encouraging a significant back-pressure in the device which is undesirable as it adversely affects the sensitivity and efficiency of the device.

It is, therefore, an object of the present invention to provide a reliable and easily adapted device which is more sensitive than the prior art for measuring lung function.

### Summary of the Invention

In its broadest aspect, the invention concerns the advantageous manipulation or redirection of an air-flow.

According to a first aspect of the present invention there is therefore provided an air-flow valve, for use in a device for measuring air-flow, having associated therewith at least one rotary blade means comprising a planar surface for detecting air-flow thereabout, which valve includes at least one air-directing aperture positioned at, or adjacent, the outer edge of said blade means or the outermost part of the path traced by same, said aperture being adapted to alter the direction of air-flow incident on said valve from a first direction to a second altered direction whereby said direction-altered air-flow provides a force which causes said blade means to move characterised in that said first direction is substantially parallel to the rotational axis of said blade means and said second direction is tangential to the rotational axis of said blade means, and in that said second altered direction is perpendicular to or at least towards the planar surface of said blade means.

Preferably, at least a part of said air-flow valve which is in the region of said blade means is transparent or translucent. This is favoured because a motion detecting means which relies on the transmission and detection of an infra-red or light beam can be used with said valve.

In a preferred embodiment, said blade means rotates through a predetermined sector, which sector includes the path of an infra-red or light beam, interruptable by the passage of the blade means therethrough.

Preferably, a plurality of air-directing apertures are provided in the valve about the periphery of the path traced by the blade means. More preferably still, the apertures are equi-spaced.

Preferably said valve is elongate or tube-shaped.

In a preferred form, said valve is further provided with a plurality of fins substantially adjacent each of said air-directing apertures.

In a further preferred form, said valve is further provided with a plurality of air-flow guides substantially adjacent each of said air-directing apertures, which air-flow guides are adapted to facilitate the change in air-flow direction from said first direction to said second direction.

The air-flow valve of the invention is ideally suited for use in an air-flow measuring device, which device comprises a number of components which may be detachable. Similarly, said valve may be made from a number of detachable components thus facilitating cleaning.

According to a second aspect of the present invention, there is therefore provided a device for measuring air-flow comprising:
an air inlet;
an air-flow valve, as defined hereinabove, including at least one blade means adapted to rotate in response to a force directed through the air inlet;
one or more optical transducers adapted to monitor the movement of the or each blade means ;
processing means for determining a required parameter from the output(s) of optical transducer(s); and
display means for visually representing each required parameter.

Preferably, the device is arranged so that movement of the blade means affects said transducers, ideally by interrupting an infra-red or light beam.

More preferably, said inlet includes a mouthpiece ideally including an elastomeric means so that variable mouthpieces can be attached to the device. Alternatively, said inlet includes a releasable mouthpiece.

### Brief Description of the Drawings

A particular embodiment of the present invention will now be described, by way of example, with reference to the accompanying drawings wherein:
Figure 1 shows two perspective views of the air-directing valve;
Figure 2 shows a top view of the air-directing valve;
Figure 3 shows a plan view of the air-directing valve;
Figure 4 shows a side sectional view of the air-directing valve;
Figure 5 shows sectional, side and end views of a mouthpiece;
Figure 6 shows two perspective views of the mouthpiece extension;
Figure 7 shows a perspective view of the device for measuring air-flow;
Figure 8 shows a side sectional view of the device;
Figure 9 shows a top view of the device;
Figure 10 is a schematic representation of the optical transducer, processing and display means.
Figure 11 shows a top view of an air-directing valve having fins 16;
Figure 12 shows a side sectional view of the air-directing valve of Figure 11;
Figure 13 is a perspective view of insert 18;
Figure 14 is a cross-sectional view of a guide 19;
Figure 15 is a side perspective view of a peak-flow meter incorporating insert 18.

### Description of the Preferred Embodiment

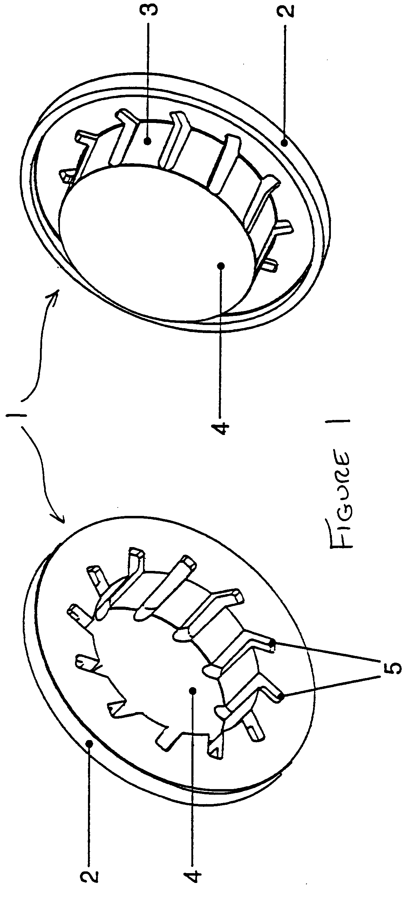

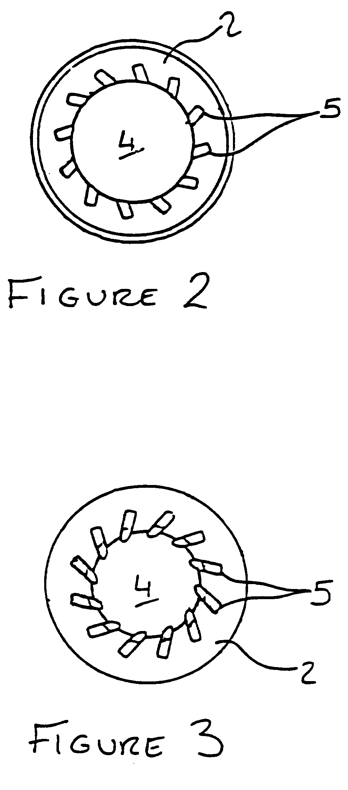

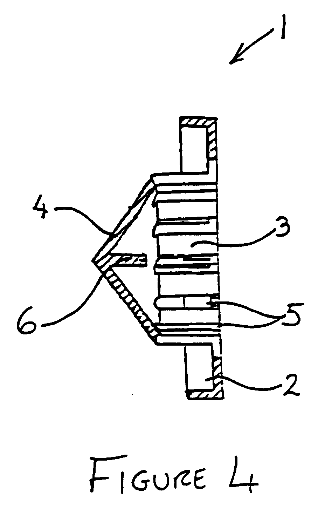

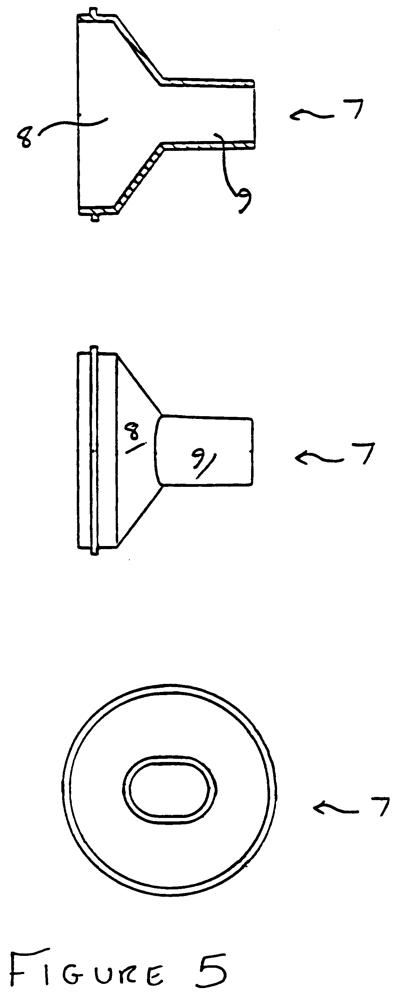

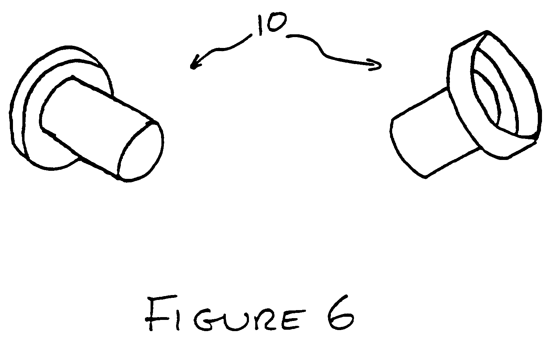

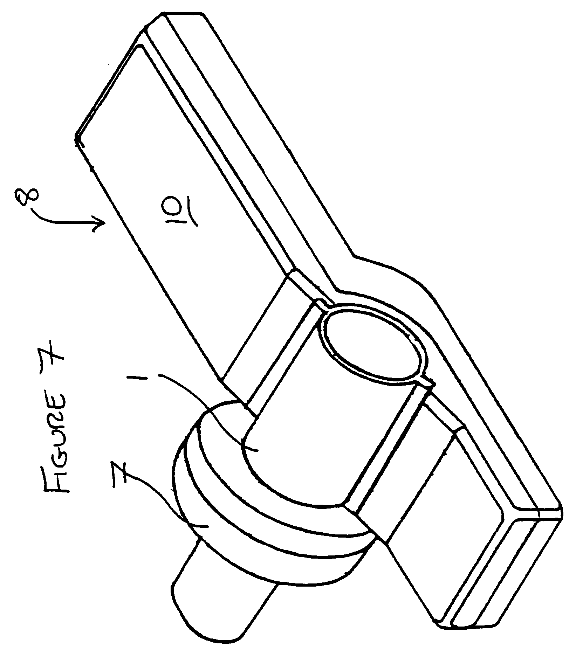

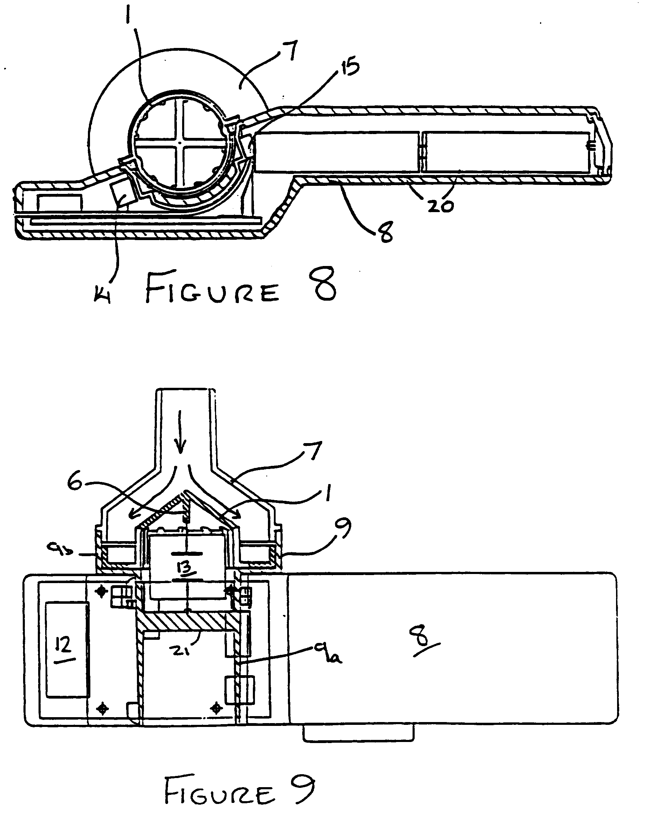

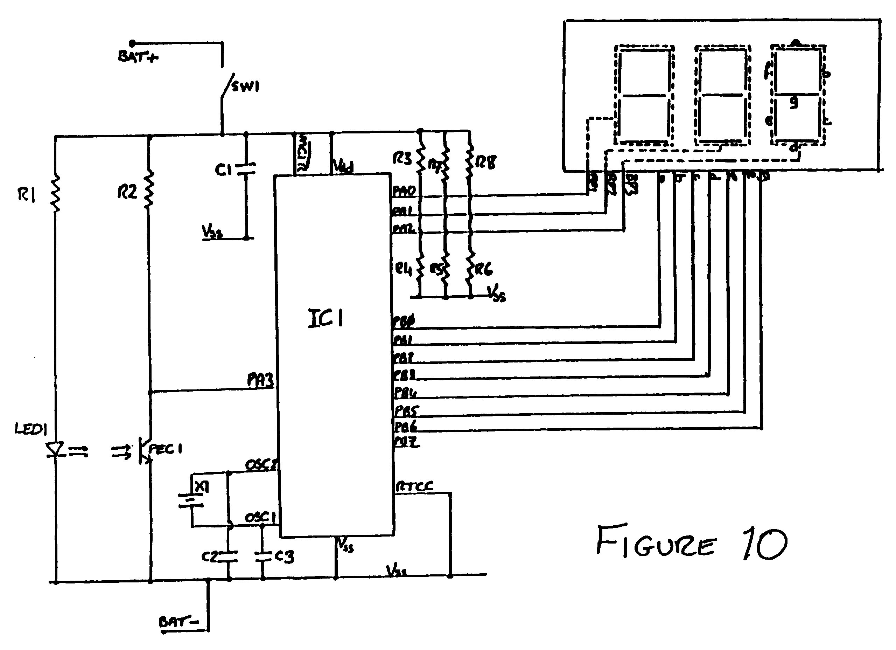

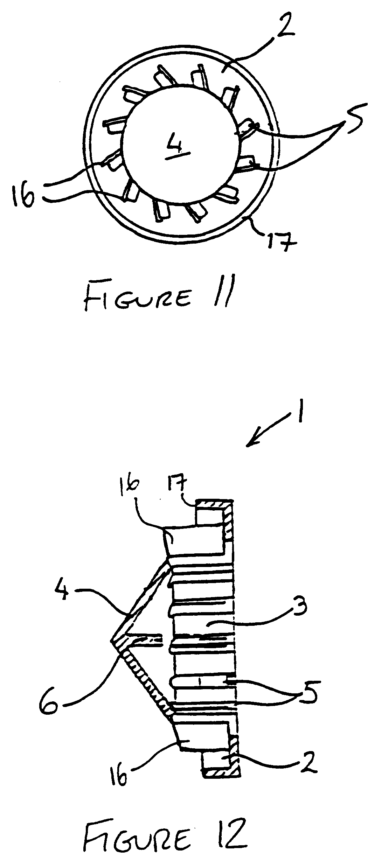

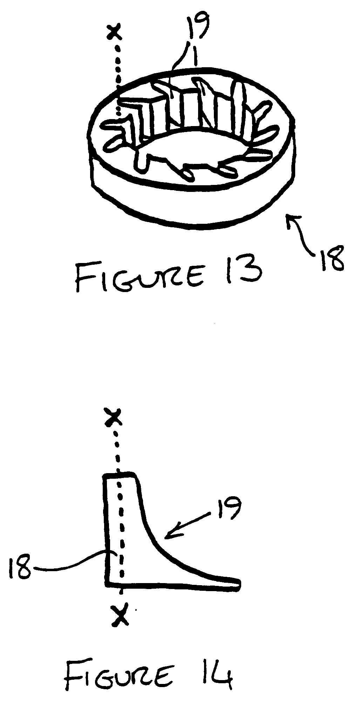

Referring to Figure 1 the air-flow valve 1 consists of a disc-shaped (or alternatively barrel-shaped) body 2 with an upstanding portion 3 of smaller diameter at its centre. The outer part of the upstanding portion has a conical cap 4.

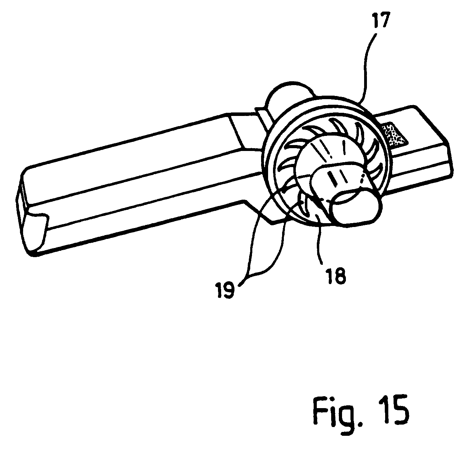

Figures 1-3 show the positioning of a series of equispaced apertures 5 around the inner part or perimeter of the upstanding portion 3. Apertures 5 are elongate in cross-section as shown in Figures 2 and 3 and ideally also angled so as to efficiently direct air-flow. In a preferred form (Figures 11 and 12), apertures 5 are extended by means of upstanding fins or flaps 16 glued in place, or integrally formed along one edge of each aperture 5. The fins may extend all the way to the rim 17 of body 2.

Figure 4 shows the provision of a blade support 6 depending inwardly from the inside of the apex of conical cap 4.

Figure 5 shows a mouthpiece 7 consisting of a funnel-shaped portion 8 integral with a narrowed portion 9 whose cross-sectional shape can be seen in the lowermost view in Figure 5.

Figure 6 shows a mouthpiece extension 10.

Figures 7-9 show an assembled device for measuring air-flow. The air-flow 1 is releasably fitted inside mouthpiece 7 and this assembly is then releasably and frictionally attached to a casing 8 by a connector 9. Connector 9 is hollow and comprises a tubular portion 9A and a disc-shaped mating portion 9B. Portion 9A is sized and shaped so as to frictionally fit a semi-circular recess provided on the upper side of casing 8. Part way along portion 9B there is provided a transverse blade mounting means 21. Casing 8 consists of two mouldings, upper moulding 10 and lower moulding 11, which releasably fit together to define a hollow casing within which replaceable batteries 20 and electronics are housed. A transparent display panel 12 is provided on the upper moulding 10 of casing 8 as shown in Figure 9.

A rectangular blade 13 is attached between blade support 6 and the blade mounting means 21 of connector 9. Alternatively, blade 13 can be integrally formed with its shaft and can have self-lubricating bearings. The blade can be any preferred shape for example a rectangular shape and it is mounted so that it is free to rotate within the air-flow valve 1. However, the position of the blade is such that when the valve 1 and mouthpiece 7 are mounted on casing 8, via connector 9, the blade is located between an emitter 14 and detector 15.

In use, a subject blows into mouthpiece 7, the exhaled air travelling in the direction of the arrows in Figure 9. The orientation of apertures 5 cause the air to be re-directed into the area enclosed by upstanding portion 3, and so impinge on the blade 13, causing it to rotate. The air-flow therefore is caused to divert through essentially 90° and so travel towards the upstanding or planar surface of blade 13.

An infra-red emitting diode 14 is positioned within casing 8 such that its infrared beam is received by an infrared sensor 15. The path of the beam is intercepted by blade 13 (a sufficient portion of valve 1 and connector 9 needs to be transparent to permit this, alternatively, suitably placed holes are provided in valve 1 and connector 9) and the periodic interruption of the infra-red beam is used to determine the frequency of rotation of the blade 13.

Microprocessor IC1 (shown in Figure 10) is used to process the output of the infra-red sensor 15 in order to drive a LCD which displays a representation of the volume of air exhaled by the subject. Appendix 1 provides suggested component values and further details of the components to be used.

A further embodiment of the invention is shown in Figures 13, 14 and 15. In that embodiment an insert 18 is provided (either attached or integrally-formed) between the upstanding portion 3 and the rim 17 of valve body 2.

The insert 18 is provided with air-flow guides 19 which correspond to the apertures 5 of the air-flow valve. These guides 19 serve to assist in the smooth air-flow between the first (incident) direction through 90° to the second direction (towards the planar surface of blade 13).

The curved shape of the guides 19 (see Figure 14 for a cross-sectional view) is, in this embodiment, preferable to causing the incident air-flow to change sharply from the first direction to the second, as is the case when insert 18 is not used. This embodiment (with insert 18 in place) is particularly effective when the incident air-flow rate is low.

The air-flow valve of the invention thus provides a means of increasing the sensitivity of a respiratory device by simply making maximum use of expired air. Re-direction of air-flow is employed in order to reduce inefficiency of existing systems.

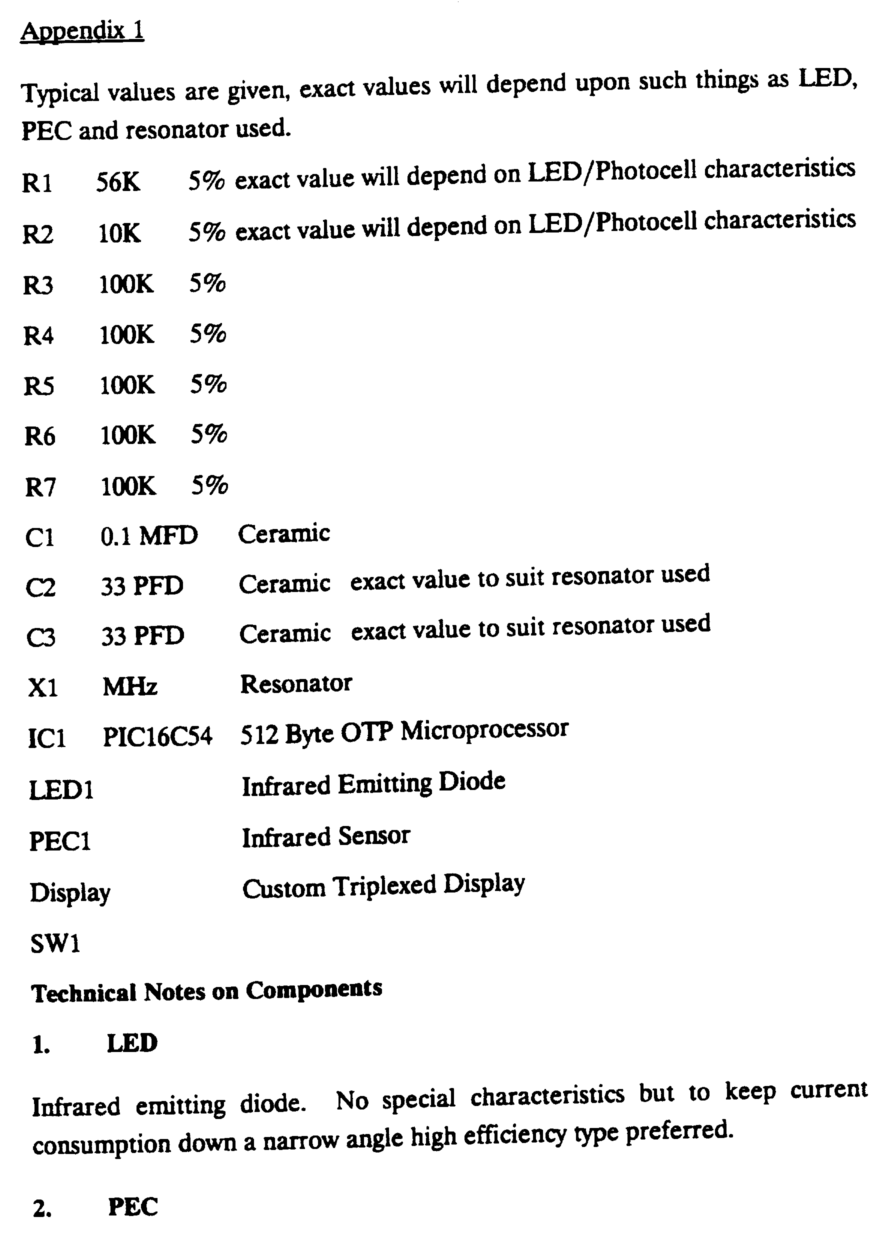

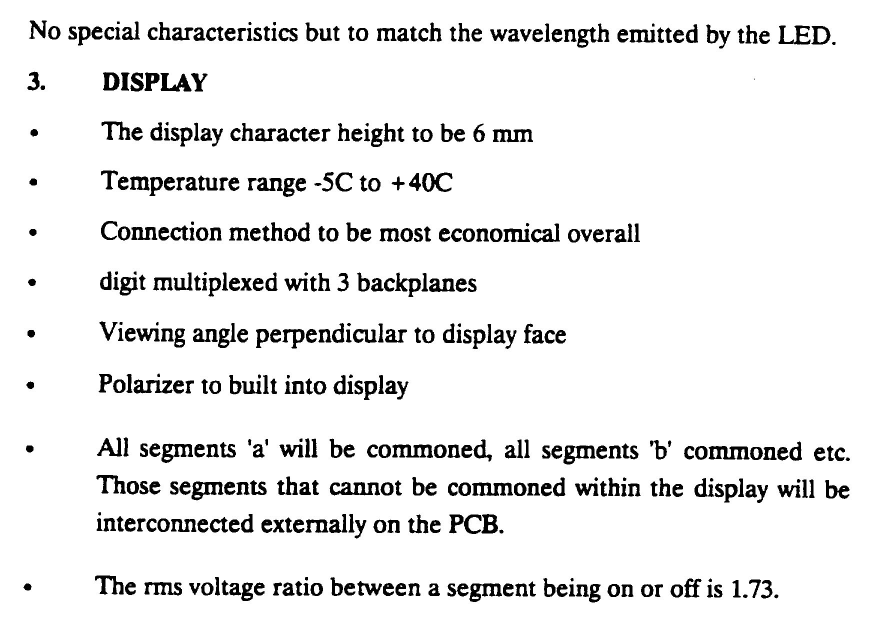

## Claims

1. An air-flow valve (1), for use in a device for measuring air-flow, having associated therewith at least one rotary blade means (13) comprising a planar surface for detecting air-flow thereabout, which valve (1) includes at least one air-directing aperture (5) positioned at, or adjacent, the outer edge of said blade means (13) or the outermost part of the path traced by same, said aperture being adapted to alter the direction of air-flow incident on said valve (1) from a first direction to a second altered direction whereby said direction-altered air-flow provides a force which causes said blade means (13) to move characterised in that said first direction is substantially parallel to the rotational axis of said blade means (13) and said second direction is tangential to the rotational axis of said blade means (13), and in that said second altered direction is perpendicular to or at least towards the planar surface of said blade means (13).

2. An air-flow valve (1) according to Claim 1 characterised in that at least part of said air-flow valve (1) which is in the region of said blade means (13) is transparent or translucent.

3. An air-flow valve (1) according to Claim 2 characterised in that said blade means (13) rotates through a predetermined sector, which sector includes the path of an infra red or light beam, interruptable by the passage of the blade means (13) therethrough.

4. An air-flow valve (1) according to any of the preceding claims characterised in that a plurality of air-directing apertures (5) are provided in the valve (1) about the periphery of the path traced by the blade means (13).

5. An air-flow valve (1) according to claim 4 characterised in that said apertures (5) are equi-spaced.

6. An air-flow valve (1) according to any of the preceding claims characterised in that said valve 1 is elongate or tube-shaped.

7. An air-flow valve according to any of claims 4 to 6 characterised in that said valve (1) is further provided with a plurality of fins (16) substantially adjacent each of said air-directing apertures (5).

8. An air-flow valve (1) according to any of claims 4 to 6 characterised in that said valve (1) is further provided with a plurality of air-flow guides (19) substantially adjacent each of said air-directing apertures (5), which air-flow guides (19) are adapted to facilitate the change in air-flow direction from said first direction to said second direction.

9. An air-flow valve (1) according to any of the preceding claims characterised in that said valve (1) comprises a number of detachable components to facilitate cleaning.

10. A device for measuring air-flow comprising:
an air inlet (7);
an air-flow valve, as defined in claim 1, including at least one blade means (13) adapted to rotate in response to a force directed through the air inlet (7);
one or more optical transducers (14,15) adapted to monitor the movement of the or each blade means (13);
processing means (IC1) for determining a required parameter from the output(s) of said optical transducer(s) (14,15); and
display means for visually representing each required parameter.

11. A device according to claim 10 characterised in that said device is arranged so that movement of the blade means (13) affects said transducers (14, 15), ideally by interrupting infra-red or light beam.

12. A device according to claim 10 of claim 11 characterised in that said inlet includes a mouthpiece (7) ideally including an elastomeric means so that variable mouthpieces (7) can be attached to the device.

13. A device according to claim 10 or claim 13 characterised in that said inlet includes a releasable mouthpiece (7).

14. A device according to any of claims 10 to 13 which device comprises a number of components which may be detachable, thus facilitating cleaning.

## Patentansprüche

1. Luftstromventil (1) zum Einsatz in einem Luftstrom-Meßgerät mit mindestens einer zugehörigen drehbaren Schaufelvorrichtung (13) zur Ermittlung des umfließenden Luftstroms, wobei das Ventil (1) mindestens eine luftleitende Öffnung (5) aufweist, die am oder neben dem äußeren Rand der genannten Schaufelvorrichtung (13) oder dem äußersten Teil des von der Schaufelvorrichtung durchlaufenen Weges angebracht ist, wobei die genannte Öffnung dazu dient, den auf das genannte Ventil (1) auftreffenden Luftstrom von einer ersten Richtung in eine zweite geänderte Richtung zu ändern, wobei der genannte richtungsgeänderte Luftstrom eine Kraft bietet, welche die Schaufel (13) dreht, dadurch gekennzeichnet, daß die genannte erste Richtung weitaus parallel zur Rotationsachse der genannten Schaufelvorrichtung (13) verläuft und die genannte zweite Richtung tangential zur Rotationsachse der genannten Schaufelvorrichtung (13) verläuft und wobei die genannte zweite geänderte Richtung senkrecht zu oder mindestens in Richtung der ebenen Fläche der genannten Schaufelvorrichtung (13) angeordnet ist.

2. Luftstromventil (1) gemäß Anspruch 1, dadurch gekennzeichnet, daß mindestens ein Teil des genannten Luftstromventils (1), der sich im Bereich der genannten Schaufelvorrichtung (13) befindet, transparent oder durchsichtig ist.

3. Luftstromventil (1) gemäß Anspruch 2, dadurch gekennzeichnet, daß sich die genannte Schaufelvorrichtung (13) durch einen vorbestimmten Bereich dreht, wobei ein Infrarot- oder Lichtstrahl durch diesen Bereich führt, der durch die Bewegung der Schaufel (13) unterbrochen wird.

4. Luftstromventil (1) gemäß jedem der vorstehenden Ansprüche, dadurch gekennzeichnet, daß mehrere luftleitende Öffnungen (5) im Ventil (1) um den Umfang des von der Schaufelvorrichtung (13) zurückgelegten Weges vorgesehen sind.

5. Luftstromventil (1) gemäß Anspruch 4, dadurch gekennzeichnet, daß die genannten Öffnungen (5) in gleichmäßigen Abstand zueinander liegen.

6. Luftstromventil (1) gemäß jedem der vorstehenden Ansprüche, dadurch gekennzeichnet, daß das genannte Ventil länglich oder röhrenförmig.

7. Luftstromventil (1) gemäß Anspruch 4 bis 6, dadurch gekennzeichnet, daß das Ventil (1) weiterhin mehrere Rippen (16) neben jeder der genannten luftleitenden Öffnungen (5) aufweist.

8. Luftstromventil (1) gemäß Anspruch 4 bis 6, dadurch gekennzeichnet, daß das genannte Ventil (1) weiterhin mehrere Luftstromführungen (19) aufweist, die weitaus neben jedem der genannten luftleitenden Öffnungen (5) angeordnet sind, wobei die Luftstromführungen (19) dazu ausgebildet sind, die Änderung der Luftstromrichtung von der genannten ersten Richtung zur genannten zweiten Richtung zu ermöglichen.

9. Luftstromventil (1) gemäß jedem der vorstehenden Ansprüche, dadurch gekennzeichnet, daß das Ventil (1) aus einer Anzahl Teile besteht, die zur Reinigung abnehmbar sind.

10. Vorrichtung zur Messung des Luftflusses einschließlich:
einem Lufteinlaß (7);
einem Luftstromventil, wie in Anspruch 1 beschrieben, einschließlich mindestens einer Schaufelvorrichtung (13), die sich durch eine Kraft, die durch den Lufteinlaß (7) gelenkt wird, dreht;
einem oder mehreren optischen Meßwandlern (14, 15), die zur Überwachung der Bewegung der einzigen oder der einzelnen Schaufelvorrichtung(en) (13) dient;
einer Verarbeitungsvorrichtung (IC1) zur Ermittlung eines benötigten Parameters vom (von den) Ausgangssignal(en) des (der) optischen Meßwandler(s) (14, 15); und
einer Anzeige zur visuellen Darstellung jedes benötigten Parameters.

11. Vorrichtung gemäß Anspruch 10, dadurch gekennzeichnet, daß die Vorrichtung so angeordnet ist, daß die Bewegung einer Schaufelvorrichtung (13) den genannten Meßwandler (14, 15) beeinflußt und idealerweise durch Unterbrechen eines Infrarot- oder Lichtstrahls.

12. Vorrichtung gemäß Anspruch 10 oder 11, dadurch gekennzeichnet, daß der genannte Einlaß ein Mundstück (7) aufweist mit idealerweise einer elastischen Vorrichtung, so daß das verstellbare Mundstück (7) am Gerät befestigt werden kann.

13. Vorrichtung gemäß Anspruch 10 oder 12, dadurch gekennzeichnet, daß der genannte Einlaß ein lösbares Mundstück (7) aufweist.

14. Vorrichtung gemäß Anspruch 10 oder 13, dadurch gekennzeichnet, daß die Vorrichtung aus einer Anzahl Teile besteht, die zur Reinigung abnehmbar sind.

## Revendications

1. Une vanne de débit d'air (1), destinée à être utilisée au sein d'un dispositif de mesure du débit d'air, auquel est associé au moins un dispositif d'hélice tournante (13) comprenant une surface plane, pour détecter le débit d'air local, laquelle vanne (1) comprend au moins une ouverture de direction d'air (5) située sur, ou à proximité du bord externe dudit dispositif d'hélice (13) ou bien au point le plus éloigné de la trajectoire tracée par celui-ci, ladite ouverture étant conçue pour changer la direction du flux d'air incident sur ladite vanne (1) d'une direction initiale en une deuxième direction modifiée et par quoi ledit flux d'air de direction modifiée exerce une force qui provoque le déplacement dudit dispositif d'hélice (13) et caractérisé en ce que ladite direction initiale est pratiquement parallèle à l'axe de rotation dudit dispositif d'hélice (13) et que ladite deuxième direction est tangentielle à l'axe de rotation dudit dispositif d'hélice (13), et en ce que la deuxième direction modifiée est perpendiculaire à, ou au moins relativement à, la surface plane dudit dispositif d'hélice (13).

2. Une vanne de débit d'air (1) selon la Revendication 1 caractérisée par le fait qu'au moins une partie de ladite vanne de débit d'air (1) qui est située dans la région dudit dispositif d'hélice (13) est transparente ou translucide.

3. Une vanne de débit d'air (1) selon la Revendication 2 caractérisée en ce que ledit dispositif d'hélice (13) tourne dans un secteur prédéterminé, lequel secteur englobe le trajet d'un faisceau infrarouge ou lumineux, qui peut être interrompu par le passage du dispositif d'hélice (13) à travers celui-ci.

4. Une vanne de débit d'air (1) selon l'une quelconque des Revendications précédentes et caractérisée par le fait qu'une pluralité d'ouvertures de direction d'air (5) sont pratiquées dans la vanne (1) au niveau de la périphérie de la trajectoire tracée par le dispositif d'hélice (13).

5. Une vanne de débit d'air (1) selon la revendication 4 caractérisée en ce que lesdites ouvertures (5) sont équidistantes.

6. Une vanne de débit d'air (1) selon l'une quelconque des Revendications précédentes et caractérisée en ce que ladite vanne 1 est de forme allongée ou tubulaire.

7. Une vanne de débit d'air (1) selon n'importe lesquelles des Revendications 4 à 6 caractérisée en ce que ladite vanne est en outre munie d'une pluralité d'ailettes (16) chacune étant pratiquement adjacente auxdites ouvertures de direction d'air (5).

8. Une vanne de débit d'air (1) selon n'importe lesquelles des Revendications 4 à 6 caractérisée en ce que ladite valve (1) est en outre munie d'une pluralité de conduites guides d'air (19) chacune étant pratiquement adjacente auxdites ouvertures de direction d'air (5), lesquelles conduites guides d'air (19) sont conçues pour faciliter le changement de direction du flux d'air de ladite direction initiale en ladite deuxième direction.

9. Une vanne de débit d'air (1) selon l'une quelconque des revendications précédentes caractérisée en ce que ladite valve (1) comporte un certain nombre d'éléments détachables pour en faciliter le nettoyage.

10. Un dispositif débimétrique de flux d'air comportant:
une entrée d'air (7);
une vanne de débit d'air, telle que définie dans la Revendication 1, comportant au moins un dispositif d'hélice (13) conçu pour tourner sous l'effet d'une force dirigée à travers l'entrée d'air (7);
un ou plusieurs transducteurs optiques (14, 15) conçus pour suivre le mouvement du, ou de chaque, dispositif d'hélice (13);
des moyens de traitement (IC1) pour déterminer un paramètre requis à partir du signal (des signaux) sortant dudit (desdits) transducteur(s) optique(s) (14, 15); et
des moyens d'affichage pour représenter visuellement chaque paramètre requis.

11. Un dispositif selon la Revendication 10 caractérisé en ce que ledit dispositif est conçu de façon à ce que le mouvement du dispositif d'hélice (13) agisse sur lesdits transducteurs (14, 15), de préférence par l'interruption d'un faisceau infrarouge ou lumineux.

12. Un dispositif selon les Revendications 10 ou 11 caractérisé en ce que ladite entrée d'air est équipée d'un embout buccal (7) comportant de préférence des éléments élastomeriques permettant de fixer plusieurs embouts buccaux (7) au dispositif.

13. Un dispositif selon la Revendication 10 ou la Revendication 13 caractérisé en ce que ladite entrée d'air est équipée d'un embout buccal (7) amovible.

14. Un dispositif selon n'importe quelle des Revendications 10 à 13 qui comporte un certain nombre d'éléments qui peuvent être détachables, facilitant ainsi le nettoyage.
